# EUROPEAN PATENT APPLICATION

(11) **EP 4 570 268 A1**
(43) Date of publication of application: **18.06.2025**
(21) Application number: 23867841.1
(22) Date of filing: 22.06.2023
(51) Int. Cl.: A61K 47/36, A61K 9/06, A61K 31/728, A61K 47/22, A61L 27/20, A61P 19/02, A61P 29/00

(54) **GEL-FORMING MATERIAL AND GEL COMPOSITION**

(30) Priority: 20.09.2022 JP 2022148868
(71) Applicant: Kewpie Corporation, Tokyo 150-0002 (JP)
(72) Inventor: FUJIKAWA Shunichi, Chofu-shi, Tokyo 182-0002 (JP)
(74) Representative: Winter, Brandl - Partnerschaft mbB
(86) International application number: PCT/JP2023/023095
(87) International publication number: WO 2024/062713

(57) **Abstract**

The present invention relates to a gel-forming material including: at least one component A selected from the group consisting of modified hyaluronic acid having an amino group and an average molecular weight of 10,000 or more and a salt thereof; and a genipin.

## Description

### Technical Field

The present invention relates to a gel-forming material and a gel composition.

### Background Art

The indwelled material gelled in situ can be used as a medical material that can be administered by injection into the body. Therefore, it is considered that minimally invasive treatment can be performed, and thus attention is paid in the fields of drug carriers and tissue engineering. For a material that gels in situ, in addition to gelling in a relatively short time in response to changes in temperature, pH and chemicals, biocompatibility and safety are also important issues.

Among various gel-forming materials, hyaluronic acid is excellent in biocompatibility and safety to the human body. As a composition for obtaining a hydrogel using hyaluronic acid, for example, Patent Literature 1 discloses a hyaluronic acid derivative having a specific structure and a crosslinked body thereof as those that can be used for treatment of cytokine related inflammation.

### Citation List

### Patent Literature

Patent Literature 1: Japanese Unexamined Patent Publication No. 2016-518464

### Summary of Invention

### Technical Problem

The present invention provides a gel-forming material and a gel composition that can be gelled in a short time under a condition of 37°C using hyaluronic acid. Such a gel-forming material and gel composition are suitable for a medical material that needs to be administered into a living body by injection and gelled in situ.

An object of one aspect of the present invention is to provide a gel-forming material capable of forming a gel in a short time under a condition of 37°C. An object of one aspect of the present invention is to provide a novel in-situ gel-forming material.

### Solution to Problem

The present inventors have found that a gel-forming material including at least one component A selected from the group consisting of modified hyaluronic acid having an amino group and an average molecular weight of 10,000 or more and a salt thereof and genipin can solve the above problems, and have completed the present invention.

That is, the present invention relates to, for example, the following inventions.
[1] A gel-forming material including: at least one component A selected from the group consisting of modified hyaluronic acid having an amino group and an average molecular weight of 10,000 or more and a salt thereof; and a genipin.
[2] The gel-forming material according to [1], wherein the amino group modification rate of the component A is 5% or more and 70% or less.
[3] The gel-forming material according to [1] or [2], wherein the component A has an average molecular weight of 10,000 or more and 1,500,000 or less.
[4] An in-situ gel-forming material including at least one selected from the group consisting of modified hyaluronic acid having an amino group and an average molecular weight of 10,000 or more and a salt thereof.
[5] An in-situ gel-forming material including genipin.
[6] A gel composition wherein at least one component A selected from the group consisting of modified hyaluronic acid having an amino group and an average molecular weight of 10,000 or more and a salt thereof is cross-linked with genipin.

### Advantageous Effects of Invention

According to the present invention, it is possible to provide a gel-forming material capable of forming a gel in a short time under the condition of 37°C. According to the present invention, it is also possible to provide a novel in-situ gel-forming material.

### Description of Embodiments

Hereinafter, embodiments for carrying out the present invention will be described in detail. However, the present invention is not limited to the following embodiments.

### <Features of Present Invention>

### (Gel-forming Material)

The present invention is characterized by providing a gel-forming material including: at least one component A selected from the group consisting of modified hyaluronic acid having an amino group and an average molecular weight of 10,000 or more and a salt thereof; and a genipin. The gel-forming material of the present invention can be gelled in a short time within 1 hour under the condition of 37°C, and can be suitably used for a medical material that needs to be gelled in situ.

### <Gel-forming Material>

The "gel-forming material" in the present specification is a material containing one or two or more the components capable of forming a gel composition by reaction with other components. The gel-forming material of the present invention may form a gel composition within 1 hour from the start of reaction when reacted with other components at 37°C.

The gel-forming material of the present invention includes at least one component A selected from the group consisting of modified hyaluronic acid having an amino group introduced therein and a salt thereof, and genipin.

### <Gel Formation in situ>

Since the gel-forming material of the present invention can form a gel in a short time of 1 hour or less under the condition of 37°C, it is suitably used as an in-situ gel-forming material. The "in-situ gel-forming material" is a gel-forming material capable of forming a gel at a target location.

The gel-forming material of the present invention can be sterilized in a state before gel formation (for example, in a solution state). Therefore, sterilization in a state after gelation is not necessarily required, and it is possible to form a gel by administering the sterilized gel-forming material to a target location of a living body.

### <Component A>

The component A is a compound in which a functional group having an amino group (-NH₂) is introduced into at least a part of hyaluronic acid or a salt thereof. However, those obtained by deacetylating the acetyl group of N-acetylglucosamine, which is a part of hyaluronic acid, to form an amino group are excluded. The gel-forming material may contain one or two or more the component A.

### (Component A Structure 1)

The component A may be a compound in which at least some of the functional groups contained in some of the disaccharide units among the repeating units of the disaccharide units constituting hyaluronic acid are substituted with a functional group having an amino group, or a salt thereof. The component A may be a compound or a salt thereof in which at least one group selected from the group consisting of a hydroxy group at the C4 position and a hydroxy group at the C6 position of N-acetylglucosamine constituting hyaluronic acid, and a hydroxy group at the C2 position, a hydroxy group at the C3 position, and -OH in a carboxy group at the C6 position of glucuronic acid constituting hyaluronic acid is substituted with a functional group having an amino group.

### (Component A Structure 2)

The component A may be, for example, a compound represented by the following formula (1) or a salt thereof.

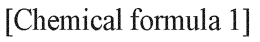

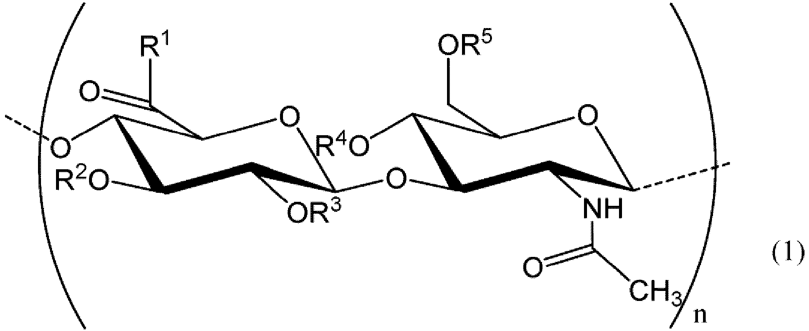

In the formula (1), R¹ represents a functional group having -OH or an amino group, and R² to R⁵ independently represent a hydrogen atom or a functional group having an amino group. n represents a number of 1 or more and 7,500 or less.

### (Number and Substitution Position of Functional Groups Having Amino Group in Formula (1))

In the formula (1), at least one of R¹ to R⁵ is a functional group having an amino group. In the formula (1), 1 to 4, 1 to 3, or 1 to 2 of R¹ to R⁵ may be a functional group having an amino group, and any one of R¹ to R⁵ may be a functional group having an amino group. In the formula (1), R¹ may be a functional group having an amino group, and all of R² to R⁵ may be a hydrogen atom.

### (Component A Structure 3)

The component A may be, for example, a compound represented by the following formula (1a) or a salt thereof.

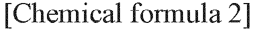

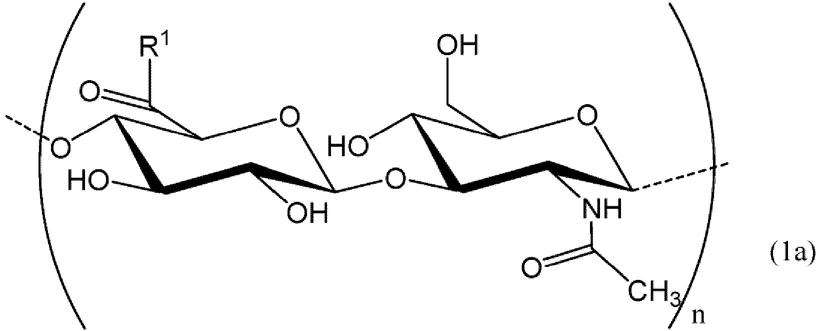

In the formula (1a), R¹ and n have the same meaning as R¹ and n in the formula (1), respectively.

### (Structure of Functional Group Having Amino Group)

Examples of the amino group include an amino group bonded to a hydrocarbon group and an amino group in a hydrazide group. The functional group having an amino group may be a group represented by formula (I): -X¹-R-NH₂.

### (Structure 1 of R in Formula (I))

R represents a divalent group, and is a group linking X¹ and NH₂. The divalent group may contain a hydrocarbon group. The hydrocarbon group may be linear or branched. The hydrocarbon group may be, for example, an alkylene group. The number of carbon atoms of the hydrocarbon group and the alkylene group may be, for example, 1 or more, or 2 or more, and may be 10 or less, 8 or less, 6 or less, 4 or less, or 3 or less. R may contain a functional group other than a hydrocarbon group. The functional group other than the hydrocarbon group may be, for example, -CO-NH-.

### (Structure 2 of R in Formula (I))

R may be a group represented by -R^{b}-R^{a}-R^{c}-. R^{a} represents an alkylene group, and R^{b} and R^{c} each independently represent a single bond or * 1-CO-NH-*2. Here, *1 represents a binding site with R^{a}, and *2 represents a binding site with X¹ or an amino group. R may contain an alkylene group having 2 or more carbon atoms. When R contains an alkylene group having 2 or more carbon atoms, the elasticity of the resulting gel composition is more excellent. When R contains an alkylene group having 4 or less carbon atoms, solubility in a solvent such as water or PBS is more excellent.

### (Structure of X¹ in Formula (I))

X¹ may be -NH- or -O- when the bonding point is R¹. X¹ may be -(C=O)- or a single bond when the bonding point is R² to R⁵.

### (Specific Example of Functional Group Having Amino Group)

When the bonding point is R¹, the functional group having an amino group may be -NH-CH₂-CH₂-NH₂, -NH-NH-CO-CH₂-CH₂-CH₂-CH₂-CO-NH-NH₂, -NH-CH₂-NH₂, -NH-CH₂-CH₂-CH₂-NH₂, or -NH-CH₂-CH₂-CH₂-CH₂-NH₂. When the bonding point is R² to R⁵, the functional group having an amino group may be -O-CH₂-CH₂-NH₂, - (C=O)-CH₂-NH₂, or -CH₂-CH₂-NH₂.

### (Salt of Modified Hyaluronic Acid)

The salt of modified hyaluronic acid may be a pharmaceutically acceptable salt. Examples of the modified hyaluronic acid salt include alkali metal salts such as a sodium salt and a potassium salt, and ammonium salts.

### (Suitable Upper Limit of Average Molecular Weight of Component A)

The average molecular weight of the component A may be 1,500,000 or less, 1,400,000 or less, 1,300,000 or less, 1,200,000 or less, 1,100,000 or less, or 1,000,000 or less. When the average molecular weight of the component A is 1,000,000 or less, solubility in a solvent such as water or PBS is further excellent.

### (Suitable Lower Limit of Average Molecular Weight of Component A)

The average molecular weight of the component A is 10,000 or more, and may be 20,000 or more, 30,000 or more, 40,000 or more, 50,000 or more, 60,000 or more, 70,000 or more, 80,000 or more, 90,000 or more, or 100,000 or more. When the average molecular weight of the component A is 10,000 or more, the elasticity of the resulting gel composition is further excellent.

### (Suitable Numerical Range of Average Molecular Weight of Component A)

The average molecular weight of the component A is preferably 10,000 or more and 1,500,000 or less, or 100,000 or more and 1,000,000 or less, and more preferably 100,000 or more and 800,000 or less, from the viewpoint of further excellent solubility in a solvent such as water or PBS.

### (Method for Measuring Average Molecular Weight)

The average molecular weight can be measured by the following method.

First, a plurality of (purified) hyaluronic acids (reference substances) having known molecular weights are subjected to liquid chromatography analysis using a gel filtration column, and a calibration curve is prepared from the retention times of the hyaluronic acids. Similarly, the molecular weight of the component A can be determined by subjecting the component A to be measured to liquid chromatography analysis and determining the molecular weight using the prepared calibration curve.

### (Measuring Device and Measuring Conditions for Average Molecular Weight)

A 2690 separation module manufactured by Nihon Waters K.K. is used as a liquid chromatography analyzer. As a photodiode array, a 996 photodiode array manufactured by Nihon Waters K.K. is used. As the column, one TSK guard column PWXL (manufactured by Tosoh Corporation) and two TSK gel GMPW (manufactured by Tosoh Corporation) connected in series in the described order are used. In the measurement of the average molecular weight, conditions of a column temperature of 40°C, a measurement wavelength of 210 nm, a flow rate of 0.8 mL/min, a sample injection amount of 20 µL, an analysis time of 40 minutes, and a mobile phase of a 0.003 mol/L phosphate buffer - 0.15 mol/L NaCl (pH 7.0) are used. Details of other test conditions may be as described in Examples described later.

### (Definition of Amino Group Modification Rate)

The amino group modification rate of the component A means the number of amino groups contained in one unit, where the one unit is a disaccharide unit constituting hyaluronic acid, and specifically refers to the rate (%) of the number of amino groups contained per the one unit to the one unit when the one unit is 100%. In the present specification, the "disaccharide unit constituting hyaluronic acid" refers to one unit constituted by disaccharide (glucuronic acid and N-acetylglucosamine) that constitute hyaluronic acid and are bonded adjacent to each other.

### (Method for Measuring Amino Group Modification Rate)

The amino group modification rate can be measured using ¹H-NMR. Specific examples of the method for preparing the measurement sample and the measurement method are as described in Examples described later.

### (Lower Limit of Amino Group Modification Rate)

The amino group modification rate may be 5% or more, 6% or more, 7% or more, 8% or more, 9% or more, or 10% or more. When the amino group modification rate is 10% or more, the gel forming ability is further excellent even when the concentration of the component A in the gel-forming material is low, i.e., less than 1%. The amino group modification rate may be 15% or more, 20% or more, 25% or more, 30% or more, 35% or more, 40% or more, 45% or more, 50% or more, or 55% or more because gel formation can be performed in a shorter time.

### (Suitable Upper Limit of Amino Group Modification Rate)

The amino group modification rate may be 90% or less, 85% or less, 80% or less, 75% or less, or 70% or less. When the amino group modification rate is 70% or less, the solubility in water or a solvent such as PBS is further excellent.

### (Suitable Numerical Range of Amino Group Modification Rate)

The amino group modification rate is preferably 5% or more and 70% or less, and more preferably 10% or more and 60% or less from the viewpoint of further excellent solubility in a solvent such as water or PBS.

### (Form of Component A)

In the gel-forming material, the component A may be in a liquid state or a solid state (for example, a powder state). The gel-forming material may contain a solution containing the component A and a solvent or a powder containing the component A. Examples of the solvent include water and a buffer. Examples of the buffer include a phosphate buffer.

### (Solution Containing Component A)

In the solution containing the component A, the content of the component A may be 0.1 mg or more, 0.5 mg or more, 1 mg or more, more than 1 mg, 2 mg or more, 3 mg or more, 4 mg or more, or 5 mg or more, and may be 25 mg or less, 15 mg or less, or 10 mg or less, with respect to 1 mL of the solvent.

### (Method for Producing Component A)

The component A can be obtained, for example, by a method including a reaction step of reacting hyaluronic acid with a raw material compound in a reaction liquid containing hyaluronic acid and a raw material compound containing a group capable of reacting with a functional group in hyaluronic acid and an amino group. The method for producing the component A may further include a post-treatment step of subjecting the reactant obtained by the reaction step to a post-treatment.

### (Raw Material Compound)

The raw material compound may be a compound represented by formula (Ia): X²-R-NH₂ or a salt thereof. R in the formula (Ia) may be a group similar to R in the formula (I). In the formula (Ia), X² represents a group capable of reacting with a functional group in hyaluronic acid. Examples of the group capable of reacting with the functional group in hyaluronic acid include an amino group (-NH₂). The use amount of the raw material compound is appropriately adjusted according to the intended amino group modification rate of the component A, and the like.

### (Specific Examples of Raw Material Compound)

Examples of the raw material compound include ethylenediamine, adipic acid dihydrazide, methanediamine, 1,3-propanediamine (1,3-diaminopropane), 1,4-butanediamine, 2-aminoethanol, glycine, and 2-chloroethyldiamine.

### (Solvent in Reaction Step)

The reaction liquid may contain a solvent. Examples of the solvent include water and a mixed solution of water and a water-soluble organic solvent such as ethanol.

### (Reactant in Reaction Step)

The reaction liquid may contain a condensing agent. Examples of the condensing agent include a carbodiimide-based condensing agent. Examples of the carbodiimide-based condensing agent include 1-(3-dimethylaminopropyl)-3-ethylcarbodiimide hydrochloride (EDC·HCl). The reaction liquid may further contain a condensation aid in addition to the condensing agent. Examples of the condensation aid include 1-hydroxybenzotriazole (HOBT).

### (Post-treatment Step)

As the post-treatment step, for example, a step of precipitating the component A, a step of washing the component A, and the like may be performed. Examples of the method for precipitating the component A include a method in which an organic solvent such as ethanol is mixed with a reaction liquid obtained after the reaction step. Examples of the method of washing the component A include a method of washing a precipitate containing the component A with an aqueous ethanol solution or the like.

### <Genipin>

Genipin is a compound capable of forming a cross-linked structure by reaction with an amino group, and is a compound represented by the following formula.

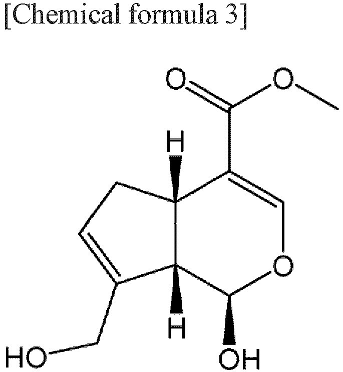

### (Mass Ratio of Genipin to Component A)

The ratio of the mass of genipin to the mass of the component A (the mass of genipin/the mass of the component A) may be 0.01 or more, 0.03 or more, or 0.05 or more. The ratio of the mass of genipin to the mass of the component A may be 1.00 or less, 0.50 or less, 0.40 or less, 0.30 or less, or 0.25 or less.

### (Form of Genipin)

In the gel-forming material, genipin may be in a liquid state or a solid state (for example, a powder state). The gel-forming material may contain a solution containing genipin and a solvent or a powder containing genipin. Examples of the solvent include water and a buffer. Examples of the buffer include a phosphate buffer.

### (Solution Containing Genipin)

In the solution containing genipin, the content of genipin may be 0.1 mg or more, 1 mg or more, or 1.5 mg or more, and may be 25 mg or less, 15 mg or less, or 10 mg or less with respect to 1 mL of the solvent.

### (Method for Obtaining Genipin)

Genipin obtained as a commercially available product can be used as it is.

### <Medicinal substance>

The gel-forming material may further contain a medicinal substance. The medicinal substance may be a water soluble medicinal substance. Examples of the medicinal substance include peptides, proteins, and genes.

### <Other Components>

The gel-forming material may contain the component A, genipin, and other components that do not correspond to the medicinal substance. Examples of other components include water, ethanol, phosphate, inorganic salts such as sodium chloride, and potassium chloride.

### <Gelling Method of Gel-forming Material>

A gel composition is formed by mixing the component A, which is the gel-forming material of the present invention, and genipin. Examples of the gelation method include a method in which a solution containing component A and a solution containing genipin are mixed and the mixture is heated to 30°C or higher, a method in which a powder containing genipin is mixed with a solution containing component A and the mixture is heated to 30°C or higher, and a method in which a powder containing component A is mixed with a solution containing genipin and the mixture is heated to 30°C or higher. The gelation method is preferably a method of mixing a solution containing the component A and a solution containing genipin from the viewpoint of suppressing generation of lumps during gel formation.

### (Temperature Condition for Gelation)

The temperature (gelation temperature) at the time of gelation of the gel-forming material may be 30°C or higher, or 35°C or higher, and may be 80°C or lower, 60°C or lower, 50°C or lower, 45°C or lower, or 40°C or lower.

### <Method for Administering Gel-forming Material>

Examples of the method for administering the gel-forming material include administration by injection. When the gel-forming material is administered into a living body by injection, the gel-forming material can be administered using a syringe including a syringe body capable of separating and storing a first component containing at least the component A and a second component containing at least genipin, and an injection needle. When the syringe is used, the first component and the second component are mixed immediately before administration into a living body (in a syringe needle of the syringe), and a gel composition is formed at a place where the first component and the second component are administered.

### <Biocompatible Gel Formation>

The gel-forming material is a cross-linking agent derived from natural (gardenia), and forms a gel composition by a reaction between genipin known to have low toxicity and the component A, so that a biocompatible gel composition can be formed.

### <Visualization of Gel Formation>

Since the gel composition formed by the reaction between the component A and genipin is colored, the formation of the gel composition can be visually confirmed. Since gel formation by the gel-forming material is visualized, it is useful in applications such as endoscopes.

### <In-situ Gel-forming Material 1>

The in-situ gel-forming material of one embodiment contains the component A described above. The in-situ gel-forming material containing the component A can form a gel in situ by the reaction with genipin described above.

### <In-situ Gel-forming Material 2>

The in-situ gel-forming material of one embodiment contains the genipin described above. The in-situ gel-forming material containing genipin can form a gel in situ by the reaction with the component A described above.

### <Gel Composition>

The gel composition of the present invention is obtained by cross-linking the component A with genipin. That is, the gel composition contains the component A and genipin, and a cross-linked structure is formed by a reaction between the component A and genipin. The gel composition is a cross-linking agent derived from natural (gardenia), and has biocompatibility because it is formed of genipin known to have low toxicity and the component A. The gel composition is colored because a cross-linked structure is formed by the reaction between the component A and genipin, and it is easy to visually confirm gelation.

### (Mode of Gel Composition)

As a specific mode of the gel composition, the specific mode of the gel-forming material described above can be applied. The gel composition may contain the above-described medicinal substance and other components. The gel composition does not exclude the inclusion of impurities inevitably contained with the preparation.

### <Method for Producing Gel Composition>

The gel composition can be obtained by a method including a step of gelling the gel-forming material described above. The gelation step can be performed, for example, by a method including mixing the above-described component A and the above-described genipin. The conditions for gelation may be as described above.

### <Use of Gel Composition>

Examples of the use of the gel composition include a local sustained release material of a medicinal substance, an adhesion-preventing material, a hemostatic material (For example, a hemostatic material for endoscopic surgery), a wound covering material, a scaffold material for cell culture, a skin filler, and a joint injection material.

### <Sustained-release Formulation>

The sustained-release formulation contains a first component containing a component A, a second component containing genipin, and a medicinal substance. The medicinal substance may be contained in one or both of the first component and the second component. The sustained release formulation contains the first component and the second component in a state before mixing. The other component described above may be included in any one or both of the first component and the second component. The sustained release formulation contains the first component and the second component in a state before mixing. By mixing the first component and the second component immediately before or at the same time of administration to a living body, a gel composition containing a medicinal substance is formed at a target site of the living body. When the target site is in a living body, the gel composition containing the medicinal substance undergoes hydrolysis or the like in the living body, and the gel composition is disintegrated, whereby the medicinal substance is gradually released.

### <Use of Component A and/or Genipin for Production of Gel-forming Material>

As one embodiment of the present invention, the use (application) of the component A and genipin for the production of a gel-forming material is provided. As one embodiment of the present invention, the use of component A for the production of an in-situ gel-forming material and the use of genipin for the production of an in-situ gel-forming material are provided. In these embodiments, the aspects of the gel-forming material described above can be applied without limitation.

### <Component A and/or Genipin Used for Gel Formation>

As one embodiment of the present invention, the component A and genipin used for gel formation are provided. As an embodiment of the present invention, the component A used for gel formation in situ and genipin used for gel formation in situ are provided. In these embodiments, the aspects of the gel-forming material described above can be applied without limitation.

### Examples

Hereinafter, the present invention will be described more specifically based on Examples and the like. However, the present invention is not limited to the following Examples.

### [Production Example 1]

### <Method for Producing Modified Hyaluronic Acid Having Amino Group (Amino Group Modification Rate: 32%)>

Hyaluronic acid (Hyaluronan HA-LQH manufactured by Kewpie Corporation, average molecular weight about 1.8 million) 1 g was dissolved in 100 mL of pure water to obtain a hyaluronic acid solution. 1-Hydroxybenzotriazole (HOBT) 1530 mg, 1920 mg of 1-(3-dimethylaminopropyl)-3-ethylcarbodiimide hydrochloride (EDC·HCl) and 13 g of adipic acid dihydrazide were added to the hyaluronic acid solution and reacted overnight. Ethanol 300 mL was slowly added to the resulting reaction liquid to precipitate the modified hyaluronic acid, and the reaction solution was washed with 100 mL of 80% ethanol 3 times to obtain an amino group-containing modified hyaluronic acid containing a modifying group derived from adipic acid dihydrazide (amino group-containing modified HA). The modification rate of the amino group-containing modified HA was 32%.

### [Production Example 2]

### <Method for Producing Modified Hyaluronic Acid Having Amino Group (Amino Group Modification Rate: 66%)>

Hyaluronic acid (Hyaluronan HA-LQ manufactured by Kewpie Corporation 0.5 g, average molecular weight: about 1.2 million) was dissolved in 50 mL of pure water to obtain a hyaluronic acid solution. 1-Hydroxybenzotriazole (HOBT) 211 mg, 240 mg of 1-(3-dimethylaminopropyl)-3-ethylcarbodiimide hydrochloride (EDC·HCl) and 166 mg of ethylenediamine dihydrochloride (EDA) were added to the hyaluronic acid solution and reacted overnight. To the resulting reaction liquid, 150 mL of ethanol was slowly added to precipitate the modified hyaluronic acid, and the reaction solution was washed with 50 mL of 80% ethanol 3 times to obtain an amino group-containing modified HA containing a modifying group derived from ethylenediamine. The modification rate of the amino group-containing modified HA was 66%.

### [Production Examples 3 to 13]

Amino group-containing modified HA was obtained in the same manner as in Production Example 1 or 2 except that the type and amount of hyaluronic acid (HA) used, the amounts of HOBT and EDC·HCl, and the type and amount of the raw material compound containing a modifying group were changed as shown in Table 1.

**[Table 1]**

| Production Example | Product Name of HA (All from Kewpie Corporation) | Molecular weight of HA | Amount of HA (g) | HOBT (mg) | EDC·HCl (mg) | Raw material compound containing modifying group | Amount of raw material compound containing modifying group (mg) |
|---|---|---|---|---|---|---|---|
| 1 | hyaluronic acid HA-LQH | 1.8 million | 1 | 1530 | 1920 | Adipic acid dihydrazide | 13000 |
| 2 | hyaluronic acid HA-LQ | 1.2 million | 0.5 | 211 | 240 | Ethylenediamine hydrochloride | 166 |
| 3 | Hyabest(S)LF-P | 300,000 | 0.5 | 27 | 30 | | 21 |
| 4 | Hyabest(S)LF-P | 300,000 | 0.5 | 53 | 60 | | 42 |
| 5 | Hyabest(S)LF-P | 300,000 | 0.5 | 106 | 120 | | 84 |
| 6 | Hyabest(S)LF-P | 300,000 | 0.5 | 211 | 240 | | 166 |
| 7 | Hyabest(S)LF-P | 300,000 | 0.5 | 422 | 480 | | 332 |
| 8 | Hyalo-Oligo | 6000 | 0.5 | 211 | 240 | | 166 |
| 9 | Hyabest(J) | 800,000 | 0.5 | 53 | 60 | | 42 |
| 10 | Hyabest(J) | 800,000 | 0.5 | 27 | 30 | | 21 |
| 11 | hyaluronic acid HA-LQH | 1.6 million | 0.5 | 27 | 30 | | 21 |
| 12 | Hyabest(S)LF-P | 300,000 | 0.5 | 106 | 120 | 1,3-Propanediamine | 46 |
| 13 | Hyabest(S)LF-P | 300,000 | 0.5 | 106 | 120 | 1,4-Butanediamine | 55 |

### <Method for Measuring Amino Group Modification Rate>

(Sample Preparation) A sample 7 mg and 1 mg of sodium 4,4-dimethyl-4-silapentanesulfonate (DSS) as an internal standard substance were dissolved in 0.7 mL of heavy water, transferred to an NMR sample tube, and capped.

### (Measurement Conditions)

Apparatus: VarianNMRsystem400NB type (Varian Technologies Japan Limited)
Observation frequency: 400 MHz
Temperature: 30°C
Reference: DSS (0 ppm)
Number of integrations: 64 times

### (Analysis Method)

In ¹H-NMR spectra, a peak at 2.0 ppm derived from CH₃ of an N-acetyl group of amino group-containing modified hyaluronic acid, a peak at 1.65 ppm derived from a modifying group derived from adipic acid dihydrazide, and a peak at 3.2 ppm derived from CH₂ of a modifying group derived from ethylenediamine were integrated. The amino group modification rate was determined from the integral value by the following formula. Amino group modification rate (%) = (peak integral value of modifying group of amino group-containing modified hyaluronic acid/2)/(peak integral value at 2.0 ppm/3) × 100

### <Method for Measuring Average Molecular Weight of Amino Group-containing Modified Hyaluronic Acid>

The obtained amino group-containing modified hyaluronic acid was dissolved in a mobile phase at a concentration of 0.1%, and the average molecular weight was relatively measured by the following liquid chromatography analysis. Using a plurality of (purified) hyaluronic acids having known average molecular weights as reference substances, a calibration curve was prepared from their retention times, and the average molecular weight of the amino group-containing modified hyaluronic acid was calculated.
Column: TSK guard column PWXL + TSK gel GMPW × 2
Column temperature: 40°C
Measurement wavelength: 210 nm
Flow rate: 0.8 mL/min
Sample injection amount: 20 µL
Analysis time: 40 minutes
Mobile phase: 0.003 mol/L phosphate buffer - 0.15 mol/L NaCl (pH 7.0)
Photodiode array: 996 photodiode array manufactured by Nihon Waters K.K.
HPLC system: 2690 separation module manufactured by Nihon Waters K.K.

### [Test Example]

### <Method for Producing Gel Composition>

Using the amino group-containing modified hyaluronic acid of Production Examples 1 to 13 and genipin, a gel composition of Test Examples 1 to 18 was obtained. Specifically, amino group-containing modified hyaluronic acid and genipin in the amounts shown in Table 2 were dissolved in water or phosphate buffered saline (PBS) to prepare solutions adjusted to the respective temperatures. The solution was turned over together with the container and left for 1 minute, and the time point when it was confirmed that the solution remained on the place and did not flow was regarded as gelation. "Room temperature" in Table 2 means 23°C.

**[Table 2]**

| Test Example No. | Amino group-containing modified HA | | | | | Solvent | | Amino group-containing modified HA concentration in solution | Amount of genipin | Temperature at time of mixing | Gelling time |
|---|---|---|---|---|---|---|---|---|---|---|---|
| | Production Example No. | Modifying group | Modification rate | Average molecular weight | Amount | Type | Amount of solvent | | | | |
| 1 | 1 | Derived from adipic acid dihydrazide | 32% | 510,000 | 20 mg | Water | 2 mL | 10 mg/mL | 4 mg | 37°C | 40 minutes |
| 2 | 2 | Derived from ethylenediamine | 66% | 140,000 | 10 mg | Water | 1 mL | 10 mg/mL | 2 mg | 37°C | 30 minutes |
| 3 | 2 | | 66% | 140,000 | 10 mg | PBS | 1 mL | 10 mg/mL | 2 mg | 37°C | 30 minutes |
| 4 | 1 | Derived from adipic acid dihydrazide | 32% | 510,000 | 20 mg | Water | 2 mL | 10 mg/mL | 4 mg | Room temperature | 2 days |
| 5 | 3 | Derived from ethylenediamine | 22% | 250,000 | 10 mg | PBS | 1 mL | 10 mg/mL | 2 mg | 37°C | 30 minutes |
| 6 | 4 | | 29% | 250,000 | 10 mg | PBS | 1 mL | 10 mg/mL | 2 mg | 37°C | 15 minutes |
| 7 | 5 | | 41% | 250,000 | 10 mg | PBS | 1 mL | 10 mg/mL | 2 mg | 37°C | 10 minutes |
| 8 | 6 | | 56% | 240,000 | 10 mg | PBS | 1 mL | 10 mg/mL | 2 mg | 37°C | 5 minutes |
| 9 | 7 | | 64% | 210,000 | 10 mg | PBS | 1 mL | 10 mg/mL | 2 mg | 37°C | 5 minutes |
| 10 | 8 | | 37% | 5000 | 10 mg | PBS | 1 mL | 10 mg/mL | 2 mg | 37°C | Not gelled |
| 11 | 9 | | 42% | 590,000 | 25 mg | PBS | 2.5 mL | 10 mg/mL | 5 mg | 37°C | 10 minutes |
| 12 | 10 | | 27% | 780,000 | 25 mg | PBS | 2.5 mL | 10 mg/mL | 5 mg | 37°C | 15 minutes |
| 13 | 11 | | 30% | 1.3 million | 25 mg | PBS | 2.5 mL | 10 mg/mL | 5 mg | 37°C | 10 minutes |
| 14 | 7 | | 64% | 210,000 | 12.5 mg | PBS | 2.5 mL | 5 mg/mL | 5 mg | 37°C | 15 minutes |
| 15 | 7 | | 64% | 210,000 | 25 mg | PBS | 2.5 mL | 10 mg/mL | 2.5 mg | 37°C | 10 minutes |
| 16 | 7 | | 64% | 210,000 | 25 mg | PBS | 2.5 mL | 10 mg/mL | 1.25 mg | 37°C | 20 minutes |
| 17 | 12 | Derived from 1,3-propanediamine | 25% | 240,000 | 25 mg | PBS | 2.5 mL | 10 mg/mL | 5 mg | 37°C | 30 minutes |
| 18 | 13 | Derived from 1,4-butanediamine | 22% | 260,000 | 25 mg | PBS | 2.5 mL | 10 mg/mL | 5 mg | 37°C | 60 minutes |

From Test Examples 1 to 3, it can be seen that when the amino group-containing modified hyaluronic acid and genipin in Production Examples 1 and 2 were used, a gel composition was obtained within 1 hour under the condition of 37°C, and the gel composition was suitable for use in situ.

It is found from Test Example 4 that even when the temperature at the time of mixing was changed, a gel composition can be formed by using the component (A) and genipin.

Even when the amino group modification rate of the amino group-containing modified hyaluronic acid was changed, it was possible to form a gel in a short time under the condition of 37°C by using the component (A) and genipin (Test Examples 5 to 9). Even when the average molecular weight was changed, it was possible to form a gel in a short time under the condition of 37°C by using the component (A) and genipin (Test Examples 10 to 13). Even when the type of amino group-containing modified HA, the amount of amino group-containing modified HA, or the amount of genipin was changed, it was possible to form a gel in a short time under the condition of 37°C by using the component (A) and genipin (Test Examples 14 to 18).

## Claims

1. A gel-forming material comprising:
at least one component A selected from the group consisting of modified hyaluronic acid having an amino group and an average molecular weight of 10,000 or more and a salt thereof; and
a genipin.

2. The gel-forming material according to claim 1, wherein the amino group modification rate of the component A is 5% or more and 70% or less.

3. The gel-forming material according to claim 1 or 2, wherein the component A has an average molecular weight of 10,000 or more and 1,500,000 or less.

4. An in-situ gel-forming material comprising at least one selected from the group consisting of modified hyaluronic acid having an amino group and an average molecular weight of 10,000 or more and a salt thereof.

5. An in-situ gel-forming material comprising genipin.

6. A gel composition wherein at least one component A selected from the group consisting of modified hyaluronic acid having an amino group and an average molecular weight of 10,000 or more and a salt thereof is cross-linked with genipin.
